# DEMANDE DE BREVET EUROPEEN

(11) **EP 0 958 837 A1**
(43) Date de publication de la demande: **24.11.1999**
(21) Numéro de dépôt: 98870117.3
(22) Date de dépôt: 20.05.1998
(51) Int. Cl.: A61L 9/04, A61L 9/12

(54) **Dispositif diffuseur de parfum**

(71) Demandeur: Generet, Daniel, 6230 Obaix (BE)
(72) Inventeur: Generet, Daniel, 6230 Obaix (BE)
(74) Mandataire: Bairiot-Delcoux, Mariette

(57) **Abrégé**

L'invention concerne un dispositif diffuseur de parfum (1) comprenant un élément diffuseur de parfum (3;15), en matière poreuse imbibé d'un parfum et d'un produit régulateur d'évaporation. De préférence le volume V_{E} de l'élément diffuseur de parfum (3;15), le volume V_{P} du parfum et le volume V_{R} du produit régulateur d'évaporation, varient dans les proportions suivantes :
V_{E} : 20 à 150 unités de volume
V_{P} : 2 à 15 unités de volume
V_{R} : 0,5 à 5 unités de volume.
Le dispositif diffuseur de parfum comporte de préférence un élément de fixation (4) et un moyen de retenue (8).

## Description

### Domaine technique

La présente invention concerne les dispositifs diffuseurs de parfums, destinés à être mis en place dans des endroits confinés, en particulier dans des lieux publics ou privés confinés susceptibles d'être soumis à des odeurs désagréables, tels que par exemple des cabines téléphoniques ou des cabinets de toilette. Les dispositifs diffuseurs de parfum de l'invention peuvent également être installés par exemple dans des voitures et caravanes ou encore dans des poubelles, notamment des poubelles à couvercle basculant.

### Art antérieur et critique de cet art antérieur

On connaît depuis longtemps des diffuseurs de parfums pour usage domestique. Le plus souvent, ces diffuseurs comportent un réservoir de parfum, et sont commercialisés dans un emballage hermétique, qui est enlevé au moment où le consommateur souhaite utiliser le diffuseur. Chaque diffuseur est en général utilisé jusqu'à épuisement du parfum dans son réservoir, puis remplacé. Certains diffuseurs sont commercialisés avec un système de recharges, plus économique.

Différents types de support pour le parfum peuvent être utilisés, parmi lesquels le carton, et les mousses de polymères à cellules ouvertes.

On connaît, notamment par le document GB-A-2 060 392, des dispositifs diffuseurs de parfum comprenant un corps en mousse de résine synthétique ayant une structure à cellules ouvertes, suffisamment fine pour permettre au parfum liquide retenu dans les cellules de la mousse de migrer suivant une action capillaire jusqu'à la surface du dispositif.

Les endroits publics confinés et fort fréquentés, tels que par exemple les cabines téléphoniques souffrent souvent d'un manque d'hygiène.

Il est souhaitable d'utiliser dans ce type d'endroits des dispositifs diffuseurs de parfum particulièrement efficaces, dont on puisse contrôler le fonctionnement.

### But de l'invention

La présente invention a précisément pour but de proposer un diffuseur de parfum spécialement adapté pour l'utilisation dans des endroits confinés.

### Description

L'invention a pour objet un dispositif diffuseur de parfum comprenant un élément diffuseur de parfum en matière poreuse, imbibé d'un parfum et d'un produit régulateur d'évaporation. La matière poreuse peut être choisie dans le groupe comprenant les mousses de polymère à cellules ouvertes, le carton, le papier, les agglomérés de bois, les matières naturelles poreuses. Les produits régulateurs d'évaporation sont de préférence choisis dans le groupe comprenant les glycols, le terpène, les huiles essentielles, les huiles végétales, les huiles minérales, les acides gras.

De manière préférée, Je volume V_{E} de l'élément diffuseur de parfum, le volume V_{P} du parfum et le volume V_{R} du produit régulateur d'évaporation varient dans les proportions suivantes :
V_{E} : 20 à 150 unités de volume
V_{P} : 2 à 15 unités de volume
V_{R} : 0,5 à 5 unités de volume.
L'unité de volume peut être le cm³.

Dans un mode de réalisation préféré de l'invention, le dispositif diffuseur de parfum comporte un élément de fixation de l'élément diffuseur de parfum et un moyen de retenue de celui-ci. Eventuellement, l'élément diffuseur de parfum est muni d'un organe de préhension qui ne gêne pas la diffusion du parfum.

L'invention a également pour objet un élément diffuseur de parfum pour un dispositif diffuseur de parfum suivant l'invention qui est présenté dans un blister avant sa mise en place dans le dispositif.

### Description des figures

D'autres caractéristiques et avantages de l'invention apparaîtront à la lecture de la description qui suit, référence étant faite aux figures annexées.
La figure 1 illustre un premier mode de réalisation d'un dispositif diffuseur de parfum suivant l'invention, en position de fonctionnement.
La figure 2 représente un élément diffuseur de parfum, en l'occurrence en mousse de polyuréthanne.
La figure 3 illustre de manière plus détaillée un élément de fixation et un moyen de retenue pour l'élément diffuseur de parfum représenté à la figure 2.
La figure 4 illustre un élément diffuseur de parfum analogue à celui représenté à la figure 2, en position dans un blister, avant sa mise en place dans le dispositif diffuseur de parfum suivant l'invention de la figure 1.
Les figures 5 et 6 illustre des variantes d'exécution d'éléments de fixation et de moyens de retenue pour élément diffuseur de parfum suivant l'invention.
Les figures 7 à 9 sont des figures analogues respectivement aux figures 1 à 3 et illustrent une autre variante d'exécution de l'invention.

### Description détaillée de modes de réalisation

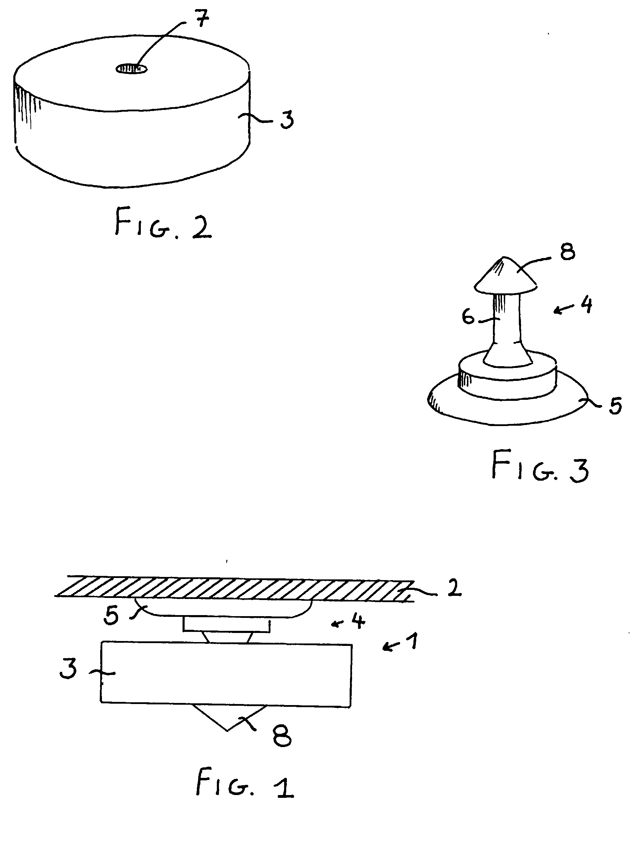

La figure 1 montre un premier mode de réalisation d'un dispositif diffuseur de parfum suivant l'invention, désigné sous la référence générale 1. Dans la figure, ce dispositif diffuseur de parfum 1 est montré déjà mis en place sur une paroi. Il est en l'occurrence placé sous une paroi horizontale 2, ici une tablette de cabine téléphonique.

Comme on le voit mieux sur les figures 2 et 3, le dispositif diffuseur de parfum comporte un élément diffuseur de parfum 3, représenté à la figure 2, dont les caractéristiques seront décrites plus loin et un élément de fixation 4 de celui-ci, dont une vue de détail est montrée à la figure 3.

Cet élément de fixation 4 est attaché sur la paroi horizontale 2 via tout moyen de fixation approprié, tel que colle, vis ou tout autre moyen adapté. Dans une forme d'exécution particulière, la base 5 de l'élément de fixation 4 est collée sur la paroi horizontale 2 par un adhésif double-face non représenté aux figures.

L'élément de fixation 4 est destiné à recevoir l'élément diffuseur de parfum 3, qui peut être enfilé sur l'axe 6, grâce à sa perforation axiale 7.

Un moyen de retenue 8 de l'élément diffuseur de parfum 3 est prévu sur l'élément de fixation 4. Lorsque le dispositif diffuseur de parfum 1 est en place comme cela est illustré à la figure 3, l'élément diffuseur de parfum 3 est placé dans une position spécialement étuduée pour une diffusion efficace du parfum. Il ne peut être retiré de l'élément de fixation 4 que par une manoeuvre volontaire.

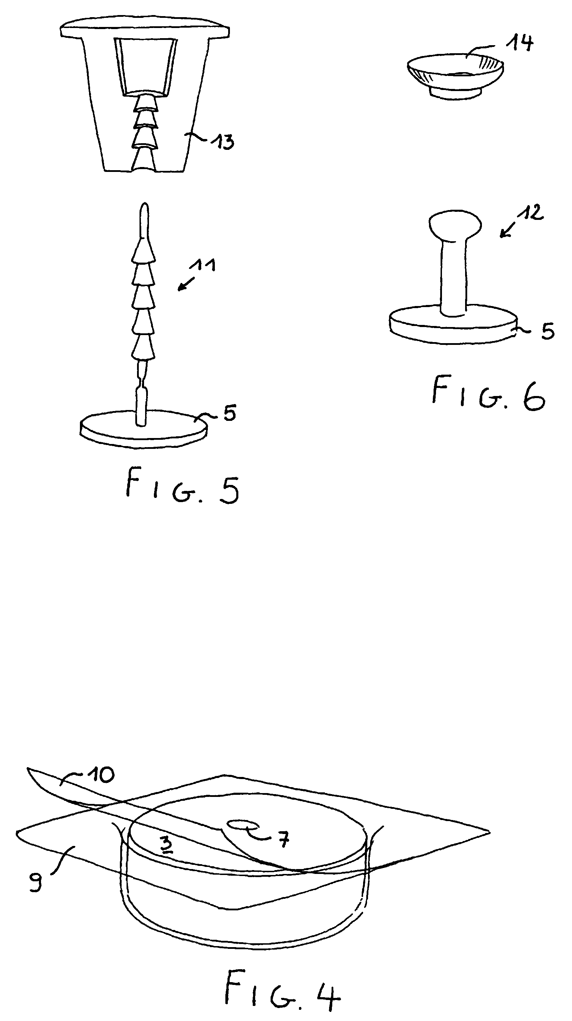

Pour la mise en place d'un élément diffuseur de parfum 3 sur un élément de fixation 4, on peut avantageusement utiliser un blister 9, représenté à la figure 4, dont l'opercule 10 peut être facilement ôté, en sorte que l'utilisateur peut aisément mettre l'élément diffuseur de parfum 3 en place sur l'axe 6 de l'élément de fixation 4 et ce sans avoir à supporter un contact direct avec l'élément diffuseur de parfum 3. Comme l'élément diffuseur de parfum 3 est par nature élastique, il franchit aisément le moyen de retenue 8.

L'élément diffuseur de parfum 3 est en matière poreuse, en l'occurrence une mousse de polyuréthanne. Il est imbibé de deux principes actifs, un parfum et un produit fixateur, dit aussi produit régulateur d'évaporation.

Dans le cadre de la présente invention, il faut comprendre que le parfum peut être constitué par toute composition à caractère parfumant et/ou désodorisant. Il est sélectionné de préférence parmi les parfums destinés à annihiler les odeurs de tabac, transpiration, décomposition de matières diverses et en général toutes les odeurs persistantes se rencontrant dans les endroits réduits et clos.

Le produit fixateur ou produit régulateur d'évaporation est un composé non évaporable destiné à retarder la circulation du parfum dans la matière poreuse et ainsi à en régulariser son évaporation.

Les produits régulateurs d'évaporation peuvent comprendre les glycols (éthylène glycol, propylène glycol), et d'autres corps à caractère gras comme les huiles végétales ou minérales (spindle, paraffine) pour autant que ces dernières soient compatibles avec le parfum utilisé.

Certaines compositions parfumantes comprenant des constituants gras et lourds tels que terpènes et huiles essentielles peuvent être utilisées dans le cadre de l'invention.

Le dispositif diffuseur de parfum 1 suivant l'invention fonctionne selon le principe de l'évaporation naturelle des constituants volatiles des parfums. Cette évaporation se produit spontanément à la surface externe de l'élément diffuseur de parfum, au contact de l'air. La partie évaporée est continuellement remplacée par du parfum frais provenant de l'intérieur de la masse de l'élément diffuseur de parfum. Ce déplacement s'opère par transfert alvéolaire ou par capillarité.

Dans le cadre de l'invention, on peut utiliser pour la fabrication de l'élément diffuseur de parfum toute matière poreuse permettant la circulation interne du parfum, notamment les mousses de polymère à cellules ouvertes (polyuréthane, polystyrène, polyacrylamide, PVC etc...), le carton ou papier, imprimé ou non, les agglomérés de bois, des matières naturelles, d'origine minérale, végétale ou animale, par exemple la pierre ponce, la pierre de lave, la cellulose, les éponges naturelles, ...

Le choix de la matière poreuse se fera en fonction de la nature du parfum, pour des raisons de compatibilité.

Les éléments diffuseurs de parfum suivant l'invention peuvent adopter une grande variété de forme sans sortir du cadre de l'invention. De même, l'élément de fixation et le moyen de retenue de l'élément diffuseur de parfum peuvent adopter toute forme appropriée adaptée à la forme de l'élément diffuseur de parfum.

Sur les figures 1 à 3, on a illustré l'invention en référence à un élément diffuseur de parfum 3 cylindrique. Il est clair que l'on peut utiliser sans diminuer l'efficacité du dispositif de l'invention des formes, sphériques, hémisphériques, cubiques, parallélépipède rectangle, pyramidale, et toute autre forme appropriée.

Les dispositifs diffuseurs de parfum de l'invention peuvent être mis en place dans toute position. Dans le cas d'un élément diffuseur de parfum de forme cylindrique tel que celui représenté à la figure 2, une mise en place sur une paroi horizontale se révèle bien adaptée. On obtient cependant un bon résultat en plaçant un dispositif dans une cabine téléphonique, sous la tablette de celle-ci. Le déplacement naturel de l'air dans le cabine suffit à assurer une activité tout à fait envisager de placer le dispositif diffuseur de parfum suivant l'invention sur un fonctionnement tout à fait satisfaisant du dispositif.

On peut aussi placer le dispositif sur une paroi verticale ou même sur une paroi oblique (comme dans le cas d'un couvercle basculant de poubelle, par exemple)

Les proportions relatives entre le volume de l'élément diffuseur de parfum, la quantité de parfum et la quantité de produit régulateur d'évaporation sont importantes pour le bon fonctionnement du dispositif diffuseur de parfum suivant l'invention.

Le volume V_{E} de l'élément diffuseur peut varier par exemple de 20 à 150 cm³, le volume V_{P} de parfum de 2 à 15 cm³ et le volume V_{R} de produit régulateur d'évaporation de 0,5 à 5 cm³. Les proportions peuvent évidemment varier en fonction du résultat désiré.

On choisira les proportions relatives en fonction notamment du volume de l'endroit confiné que l'on désire parfumer ou désodoriser et de la durée de fonctionnement que l'on souhaite impartir au dispositif diffuseur de parfum de l'invention.

Par exemple, dans le cas de cabines téléphonique, on peut désirer ne devoir effectuer le remplacement des éléments diffuseurs de parfum qu'une fois par mois.

L'élément diffuseur de parfum 3 imprégné de parfum et de régulateur d'évaporation présente un toucher gras très désagréable qui rend sa manipulation problématique. Cette caractéristique persiste même lorsque tout le parfum est évaporé. Dès lors, le dispositif de l'invention possède une protection intrinsèque contre le vol et le vandalisme particulièrement utile dans les lieux publics fort fréquentés.

Lors du retrait de l'élément diffuseur de parfum 3 de l'élément de fixation 4, en vue de son remplacement par un élément diffuseur neuf, l'utilisateur doit faire preuve de précaution et le cas échéant être muni de gants ou utiliser tout autre moyen approprié (pince, etc...).

Il n'est certainement pas exclu d'utiliser les dispositifs diffuseur de parfum suivant l'invention à un usage domestique, au quel cas l'élément diffuseur de parfum pourra avantageusement être muni d'un organe de préhension ne gênant pas la diffusion du parfum par exemple une grille à larges mailles, qui serait associé à diffuseur de parfum, placé avec celui-ci dans la coquille du blister 9 et retenu avec l'élément diffuseur de parfum par le moyen de retenue 8.

Aux figures 5 et 6, on a représenté d'autres variantes d'éléments de fixation. Contrairement à l'élément de fixation 4 représenté à la figure 3, l'élément de fixation 11 et l'élément de fixation 12 représentés respectivement aux figures 5 et 6 comportent deux parties. L'élément 11 représenté à la figure 5 fonctionne sur un principe de tenons et mortaises. Il est muni d'une pointe de type harpon.

L'élément 12 représenté à la figure 6 repose sur le principe du bouton-pression. L'un et l'autre de ces systèmes peuvent être utilisés pour la mise en place d'un élément de diffuseur de parfum 3. Lors du retrait de ce dernier après utilisation le moyen de retenue 13 ou 14 de chacun des éléments de fixation 11 ou 12 en est désolidarisé.

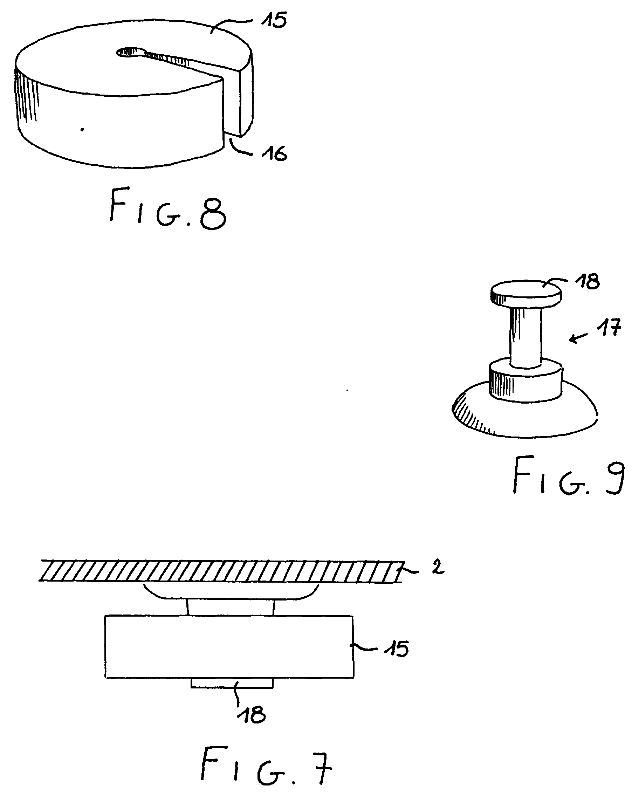

Aux figures 7 à 9, on a représenté une variante d'exécution de l'invention. Comme on le voit à la figure 8 l'élément diffuseur de parfum 15 est rigide et comporte une fente 16 qui lui permet d'être agencé sur un élément de fixation 17 comportant un moyen de retenue 18 de large diamètre qui pourrait difficilement être franchi par un élément diffuseur de parfum 3 tel que celui représenté à la figure 2. L'utilisateur effectue latéralement la mise en place et le retrait de l'élément diffuseur de parfum 15 sur l'élément de fixation 17.

Les dispositifs diffuseurs de parfum de l'invention sont très économiques, très simples et très efficaces. Ils permettent une gestion contrôlée de la diffusion de parfum. Il est important de souligner qu'ils ont été spécialement conçus pour pouvoir fonctionner sans que soit nécessaire la mise en oeuvre d'un quelconque appareil forçant la circulation d'air. Le mouvement d'air naturel des endroits confinés dans lesquels les dispositifs de l'invention sont susceptibles d'être placés suffit à leur bon fonctionnement.

## Revendications

1. Dispositif diffuseur de parfum (1) comprenant un élément diffuseur de parfum (3;15) , caractérisé en ce que l'élément diffuseur de parfum (3;15) est en matière poreuse et en ce qu'il est imbibé d'un parfum et d'un produit régulateur d'évaporation.

2. Dispositif diffuseur de parfum suivant la revendication 1, caractérisé en ce que la matière poreuse est choisie dans le groupe comprenant les mousses de polymère à cellules ouvertes, le carton, le papier, les agglomérés de bois, les matières naturelles poreuses.

3. Dispositif diffuseur de parfum suivant l'une quelconque des revendications 1 et 2, caractérisé en ce que les produits régulateurs d'évaporation sont choisis dans le groupe comprenant les glycols, le terpène, les huiles essentielles, les huiles végétales, les huiles minérales, les acides gras.

4. Dispositif diffuseur de parfum suivant l'une quelconque des revendications précédentes, caractérisé en ce que le volume V_{E} de l'élément diffuseur de parfum (3;15), le volume V_{P} du parfum et le volume V_{R} du produit régulateur d'évaporation, varient dans les proportions suivantes :
V_{E} : 20 à 150 unités de volume
V_{P} : 2 à 15 unités de volume
V_{R} : 0,5 à 5 unités de volume.

5. Dispositif diffuseur de parfum suivant la revendication 5, caractérisé en ce que l'unité de volume est le cm³.

6. Dispositif diffuseur de parfum suivant l'une quelconque des revendications précédentes, caractérisé en ce qu'il comporte un élément de fixation (4;11;12) de l'élément diffuseur de parfum (3;15) et un moyen de retenue (8;13;14;18) de celui-ci.

7. Dispositif diffuseur de parfum suivant l'une quelconque des revendications précédentes, caractérisé en ce que l'élément diffuseur de parfum (3;15) est muni d'un organe de préhension qui ne gêne pas la diffusion du parfum.

8. Elément diffuseur de parfum (3;15) pour un dispositif diffuseur de parfum suivant l'une quelconque des revendications précédentes, caractérisé en ce qu'il est présenté dans un blister (9) avant sa mise en place dans le dispositif.
